# FASCICULE DE BREVET EUROPEEN

(11) **EP 2 720 677 B1**
(45) Date de publication et mention de la délivrance du brevet: **02.12.2020**
(21) Numéro de dépôt: 12738512.8
(22) Date de dépôt: 20.06.2012
(51) Int. Cl.: A61K 8/99, A61Q 19/08

(54) **UTILISATION DE SUBSTANCES AGISSANT SUR L'IGF-1 ET/OU L'IGF-1R POUR LEUR ACTIVITE ANTI-AGE**
VERWENDUNG VON AUF IGF-1 UND/ODER IGF-1R WIRKENDEN STOFFEN FÜR ANTI-AGING-WIRKUNG
USE OF SUBSTANCES WHICH ACT ON IGF-1 AND/OR IGF-1R FOR THE ANTI-AGEING ACTIVITY THEREOF

(30) Priorité: 20.06.2011 FR 1155401
(43) Date de publication de la demande: 23.04.2014
(73) Titulaire: Societe Industrielle Limousine d'Application Biologique, 19130 Objat (FR)
(72) Inventeur: PAUFIQUE, Jean, F-19130 Objat (FR)
(74) Mandataire: Aquinov
(86) Numéro de dépôt international: PCT/FR2012/051388
(87) Numéro de publication internationale: WO 2012/175868

(56) Documents cités:
- EP-A2- 2 110 123
- WO-A2-2009/031106
- FR-A1- 2 779 058
- FR-A1- 2 828 400
- FR-A1- 2 834 462
- FR-A1- 2 887 148
- FR-A1- 2 897 266
- JP-A- 2007 210 915
- DATABASE WPI Week 200919 Thomson Scientific, London, GB; AN 2009-E59133 XP002670182, -& KR 2008 0100678 A (HYE J K) 19 novembre 2008 (2008-11-19)
- DATABASE WPI Week 200650 Thomson Scientific, London, GB; AN 2006-484887 XP002670183, -& JP 2006 176481 A (INTRON YG) 6 juillet 2006 (2006-07-06)
- DATABASE WPI Week 201065 Thomson Scientific, London, GB; AN 2010-L15251 XP002670184, -& KR 2010 0090992 A (LEE G H) 18 août 2010 (2010-08-18)
- DATABASE WPI Week 200974 Thomson Scientific, London, GB; AN 2009-Q76644 XP002670181, -& JP 2009 256270 A (MARUZEN SEIYAKU KK) 5 novembre 2009 (2009-11-05)
- DOI K: "Insulin-like growth factor-1 (IGF-1) expression promoter for preventing or treating disease associated with IGF-1 and senility, improving cognitive function and enhancing immunity, comprises extracts of Ginkgo, peppermint and rosemary", WPI / THOMSON,, vol. 2009, no. 74, 5 November 2009 (2009-11-05), XP002670181,

## Description

La présente invention concerne l'utilisation dans une composition cosmétique, d'une substance particulière comme principe actif cosmétique destiné à prévenir et/ou lutter contre le vieillissement de la peau telle que exposée dans les revendications.

L'invention vise également un procédé cosmétique pour prévenir ou lutter contre le vieillissement cutané, comprenant l'application sur la peau d'une composition comprenant au moins cette substance comme principe actif telle que exposée dans les revendications.

Dans nos sociétés, l'esthétisme et l'apparence prennent une place de plus en plus importante. Pour améliorer leur image, beaucoup de gens ont tendance à vouloir paraître jeunes le plus longtemps possible et cherchent à estomper les manifestations visibles du vieillissement de la peau.

C'est pourquoi l'objectif de la présente invention est de proposer des produits capables de lutter efficacement contre le vieillissement cutané et limiter l'apparition des effets néfastes qui en découlent. Le document EP 2110123 concerne un agent pour la croissance de fibroblastes à l'aide d'un extrait de levure.

Le vieillissement de la peau résulte d'altérations qui surviennent spontanément au cours du temps ou qui sont induites par divers facteurs externes. On distingue ainsi le vieillissement cutané intrinsèque ou chronologique qui est génétiquement programmé et le vieillissement extrinsèque ou induit qui est provoqué par diverses agressions extérieures telles que le soleil, la pollution atmosphérique, l'alimentation, l'hygiène de vie, etc. D'autres facteurs peuvent également participer comme l'altération des communications cellulaires, le vieillissement endocrinien lors de la ménopause ou encore les facteurs mécaniques de gravité terrestre.

De nombreux phénomènes moléculaires et cellulaires interviennent dans le vieillissement du tissu cutané. Certaines transformations observées sont notamment la résultante d'une modification de la sécrétion naturelle de l'organisme en hormones et en facteurs de croissance.

Pour répondre à son objectif, la présente invention vise à utiliser une substance qui puisse agir sur les mécanismes impliqués dans le vieillissement du tissu cutané, notamment sur les mécanismes aptes à lutter contre la baisse d'activité des cellules vieillissantes du tissu cutané et à réveiller biologiquement les cellules de la peau et protéger la fonction barrière cutanée.

En particulier l'invention vise l'utilisation comme principe actif cosmétique dans une composition cosmétique, au moins une substance agissant sur l'IGF-1 (« insulin-like growth factor» - facteur de croissance apparenté à l'insuline) et/ou sur l'IGF-1R (« insulin-like growth factor receptor» - récepteur au facteur de croissance apparenté à l'insuline) des cellules cutanées.

L'invention a également pour objet un procédé cosmétique pour prévenir ou lutter contre le vieillissement cutané, comprenant l'application topique sur la peau d'une composition contenant au moins une substance agissant sur l'IGF-1 et/ou sur l'IGF-1R des cellules cutanées.

Avantageusement, l'utilisation d'une substance agissant sur l'IGF-1 et/ou sur l'IGF-1R, permet notamment de diminuer les rides et les ridules et d'améliorer la fonction barrière cutanée des peaux vieillies.

D'autres avantages et caractéristiques de l'invention ressortiront de la description en détails qui va suivre.

L'objet de l'invention est l'utilisation dans une composition cosmétique d'une quantité efficace d'au moins une substance agissant sur l'IGF-1 et/ou l'IGF-1R des cellules cutanées comme principe actif cosmétique destiné à prévenir ou lutter contre le vieillissement de la peau.

Les IGF (« insulin-like growth factor » - facteurs de croissance apparentés à l'insuline), en particulier l'IGF-1 et l'IGF-2, sont des facteurs de croissance peptidiques ayant une structure chimique semblable à celle de l'insuline. Ces facteurs de croissances sont majoritairement sécrétés par le foie, mais l'IGF-1 est également sécrété par les fibroblastes du derme.

Le récepteur au facteur de croissance apparenté à l'insuline, l'IGF-1R est un récepteur transmembranaire comprenant deux sous-unités alpha et deux sous-unités béta. Il possède un site ATP (Adénosine TriPhosphate) qui est utilisé lors de son activation par phosphorylation. L'IGF-1R est exprimé dans la peau humaine au niveau des kératinocytes basaux et il est activé par l'IGF-1 sécrété par les fibroblastes dermiques par un mécanisme de phosphorylation. L'IGF-1R phosphorylé est la forme activée de l'IGF-1R.

L'intégrité de la fonctionnalité de la peau est dépendante de l'interaction synergétique entre l'IGF-1 des fibroblastes du derme et l'IGF-1R phosphorylé des kératinocytes de l'épiderme. Or, au cours du vieillissement, le taux d'IGF-1 dans les fibroblastes diminue, donc l'expression de la phosphorylation des IGF-1R dans les kératinocytes diminue aussi, ce qui entraîne une altération de la fonction barrière cutanée et l'apparition de paramètres liés au vieillissement de la peau telles des rides, une perte d'élasticité et une finesse cutanée importante. L'utilisation selon l'invention d'une substance agissant sur l'IGF-1 et/ou l'IGF-1R, permet de prévenir et/ou de lutter contre la diminution du taux d'IGF-1 et du d'augmenter la phosphorylation d'IGF-1R, dans les cellules de la peau et ainsi de prévenir et/ou de lutter contre le vieillissement de la peau.

En particulier, l'utilisation selon l'invention permet :
- de diminuer les rides et les ridules, et/ou
- d'améliorer la fonction barrière cutanée.

Préférentiellement, la substance agissant sur l'IGF-1 ou l'IGF-1R est une substance :
- augmentant l'expression des acides ribonucléiques messagers (ARNm) codant pour l'IGF-1, et/ou
- augmentant la synthèse de l'IGF-1, dans les fibroblastes de la peau, et/ou
- augmentant l'expression de la phosphorylation des IGF-1R, dans les kératinocytes épidermiques.

Cette substance est obtenue à partir d'au moins une levure.

Concernant la levure, il s'agit d'une substance agissant sur l'IGF-1 et/ou l'IGF-1R issue de la levure Candida oleophila, Selon un mode de réalisation particulièrement adapté, il s'agit d'un hydrolysat, en particulier d'un hydrolysat enzymatique d'au moins une levure.

Préférentiellement la substance agissant sur l'IGF-1 et/ou l'IGF-1R est obtenue à partir d'une levure par un procédé comprenant au moins une étape de solubilisation de la matière première et une étape d'hydrolyse enzymatique.

Selon un aspect de l'invention, la substance agissant sur l'IGF-1 et/ou l'IGF-1R des cellules cutanées est susceptible d'être sélectionnée par un test réalisé sur des cultures de cellules cutanées comprenant les étapes suivantes :
- incorporation des substances dans les milieux de culture de fibroblastes humains âgés,
- récupération des cellules et réalisation d'une PCR quantitative afin d'analyser l'expression des ARNm d'IGF-1 extraits.

Les substances capables d'agir sur l'IGF-1 et/ou sur l'IGF-1R sont destinées à être incorporées dans des compositions cosmétiques en particulier à raison de 0,01 à 20%.

La composition peut se présenter sous forme de crèmes, émulsions, huile dans-eau, eau-dans-huile ou émulsions multiples, solutions, suspensions ou encore poudres.

L'administration de la composition est préférentiellement réalisée par voie topique.

L'invention vise également spécifiquement un procédé cosmétique pour prévenir et/ou lutter contre le vieillissement cutané, comprenant l'application topique sur la peau d'une composition comprenant au moins une substance agissant sur l'IGF-1 et/ou l'IGF-1R des cellules cutanées comme principe actif cosmétique.

L'invention est à présent illustrée par des exemples non limitatifs de principes actifs utiles selon l'invention et de compositions cosmétiques les contenant.

### I. Exemple de référence:

### Exemples de substances utiles comme principes actifs selon l'invention I.1 Principe actif issu d'adzuki

Une substance utile selon l'invention peut être obtenue par la mise en œuvre d'un procédé comprenant au moins une étape de solubilisation de *Vigna angularis* (adzuki) et au moins une étape d'hydrolyse enzymatique.

Le procédé d'obtention consiste préférentiellement en la succession des étapes suivantes :
- solubilisation de Vigna angularis en poudre dans une solution aqueuse, à raison de 50 à 300g/l,
- séparation des phases soluble et insoluble par filtration, décantation ou centrifugation,
- purification par filtration, ultrafiltration, osmose inverse et/ou nanofiltration,
- hydrolyse(s) enzymatique(s) simultanée(s) et/ou successive(s),
- inactivation des activités enzymatiques résiduelles par traitement thermique,
- concentration par filtration, ultrafiltration, osmose inverse et/ou nanofiltration, et
- filtration stérilisante.

Un exemple non limitatif de principe actif issu de Vigna angularis susceptible d'être obtenu par la mise en œuvre d'un tel procédé est caractérisé par :
- un taux de matières sèches de 19,4g/l
- une teneur en protéines de 13,3g/l, soit 69% en poids de matières sèches,
- une teneur en sucres totaux de 2,8g/l,
- une teneur en cendres de 3,3g/l.

### I.2. Principe actif issu de Candida oleophila

Une substance utile selon l'invention peut être obtenue par la mise en œuvre d'un procédé comprenant au moins une étape de solubilisation du ferment *Candida oleophila* et au moins une étape d'hydrolyse enzymatique.

Le procédé d'obtention consiste préférentiellement en la succession des étapes suivantes :
- solubilisation de Candida oleophila en poudre dans une solution, à raison de 50 à 300g/l,
- séparation des phases soluble et insoluble par filtration, décantation ou centrifugation,
- hydrolyse(s) enzymatique(s) simultanée(s) et/ou successive(s),
- inactivation des activités enzymatiques résiduelles par traitement thermique,
- concentration par filtration, ultrafiltration, osmose inverse et/ou nanofiltration, et
- filtration stérilisante.

Un exemple non limitatif de principe actif issu de Candida oleophila susceptible d'être obtenu par la mise en œuvre d'un tel procédé est caractérisé par :
- un taux de matières sèches de 17,5g/l
- un pH de 7,
- une teneur en protéines de 13g/l, soit 74% en poids de matières sèches,
- une teneur en sucres totaux de 0,7g/l,
- une teneur en cendres de 4g/l

### Exemple de référence:

### I.3 Principe actif issu de Hanseniaspora opuntiae

Une substance utile selon l'invention peut être obtenue par la mise en œuvre d'un procédé comprenant au moins une étape de solubilisation du ferment *Hanseniaspora opuntiae* et au moins une étape d'hydrolyse enzymatique.

Le procédé d'obtention consiste préférentiellement en la succession des étapes suivantes :
- solubilisation de *Hanseniaspora opuntiae* en poudre dans une solution aqueuse, à raison de 50 à 300g/l,
- hydrolyse(s) enzymatique(s) simultanée(s) et/ou successive(s),
- séparation des phases soluble et insoluble par filtration, décantation ou centrifugation,
- inactivation des activités enzymatiques résiduelles par traitement thermique,
- concentration par filtration, ultrafiltration, osmose inverse et/ou nanofiltration, et
- filtration stérilisante.

### Exemple de référence:

Un exemple non limitatif de principe actif issu de Hanseniaspora opuntiae susceptible d'être obtenu par la mise en œuvre d'un tel procédé est caractérisé par :
- un taux de matières sèches de 35g/l
- un pH de 4,
- une teneur en protéines de 2.3g/l,
- une teneur en sucres totaux de 24g/l, soit 69% en poids de matières sèches,
- une teneur en cendres de 7.6g/l.

### Exemple de référence:

### I.4. Principe actif issu de Torulospora delbruekii

Une substance utile selon l'invention peut être obtenue par la mise en œuvre d'un procédé comprenant au moins une étape de solubilisation du ferment *Torulospora delbruekii* et au moins une étape d'hydrolyse enzymatique.

Le procédé d'obtention consiste préférentiellement en la succession des étapes suivantes :
- solubilisation de *Torulospora delbrueki* en poudre dans une solution aqueuse, à raison de 50 à 300g/l,
- séparation des phases soluble et insoluble par filtration, décantation ou centrifugation,
- hydrolyse enzymatique,
- inactivation des activités enzymatiques résiduelles par traitement thermique,
- filtration,
- concentration par filtration, ultrafiltration, osmose inverse et/ou nanofiltration, et
- filtration stérilisante.

### Exemple de référence:

Un exemple non limitatif de principe actif issu de *Torulospora delbrueki* susceptible d'être obtenu par la mise en œuvre d'un tel procédé est caractérisé par :
- un taux de matières sèches de 25g/l,
- une teneur en protéines de 16g/l, soit 64% en poids de matières sèches,
- une teneur en sucres totaux de 1g/l,
- une teneur en cendres de 5g/l.

### II. Exemples de compositions

Dans les exemples non limitatifs qui suivent, les compositions sont obtenues par mélange des différents composants. Les quantités indiquées sont données en pourcentage de poids.

### II.1 Exemple de composition d'anti-rides sous forme d'émulsion

La formulation est la suivante :

| | |
|---|---|
| - Isononyl isononanoate : | 5,0% |
| - Cetearyl octanoate : | 5,0% |
| - Cetyl alcool : | 4,0% |
| - Principe actif agissant sur IGF-1 et IGF-1R : | 4,0% |
| - Arachidyl alcool/Behenyl Alcool/ Arachidyl glucoside : | 3,0% |
| - PEG-100 stearate glyceryl stearate : | 2,0% |
| - Glycerol : | 2,0% |
| - Glycol palmitate : | 1,0% |
| - Myreth-3 Myristate : | 1,0% |
| - Shea butter : | 1,0% |
| - Polyacrylamide/C13-14 Isoparaffin/ Laureth-7 : | 0,75% |
| - Conservateur : | 0,7% |
| - Eau : | 70,55% |

### II.2 Exemple de composition anti-rides sous forme de gel émulsionné :

La formulation est la suivante :

| | |
|---|---|
| - Acide stéarique : | 7,0% |
| - Myristate isopropyl : | 7,0% |
| - Principe actif agissant sur IGF-1 et IGF-1R : | 4,0% |
| - Stearyl alcool/Ceteareth 20 : | 3,0% |
| - Stearyl stearate : | 1,0% |
| - Huile Minerale/dibutyl lauryl glutamide : | 1,0% |
| - Conservateur : | 0,7% |
| - Triethanolamine : | 0,3% |
| - Eau : | 76% |

### II.3 Exemple de composition raffermissante sous forme de lait :

La formulation est la suivante :

| | |
|---|---|
| - Isononyl isononanoate : | 5,0% |
| - Principe actif agissant sur IGF-1 et IGF-1R : | 4,0% |
| - Arachidyl alcool/Behenyl Alcool/ Arachidyl glucoside : | 3,0% |
| - Cetearyl alcool/ Ceteraryl glucoside : | 2,0% |
| - Polyacrylamide/C13-14 Isoparaffin/ Laureth-7 : | 2% |
| - Conservateur : | 0,7% |
| - Eau : | 83,3% |

### III. Evaluation de l'effet de substances sur l'IGF-1

### III.1 Evaluation in vitro de l'expression des ARNm codant pour l'IGF-1 sur fibroblastes humains

L'objectif de cette étude est d'évaluer la capacité des principes actifs des exemples du point I. à augmenter l'expression des ARNm codant pour l'IGF-1 dans des cultures de fibroblastes humains jeunes et âgés.

L'étude a été réalisée par PCR quantitative sur fibroblastes humains normaux dits fibroblastes jeunes (issus de donneurs < 30 ans) et dits fibroblastes âgés (issus de donneurs > 60 ans)

Le protocole opératoire est le suivant.

A J0, les fibroblastes jeunes et âgés sont ensemencés dans un milieu de culture adapté et les cellules sont ensuite incubées à 37°C.

A J2, le milieu de culture des cellules (âgées) est éliminé et remplacé par du milieu contenant les principes actifs :
- un principe actif issu de Adzuki (exemple : référence I.1) à 0,25%, 0,5% et à 1%,
- un principe actif issu de Candida oleophila (exemple I.2) à 0,25%, 0,5% et 1%, (invention)
- un principe actif issu de Hanseniaspora opuntiae (exemple : référence I.3) à 0,25%
   et 0,5%.
- Un principe actif issu de Torulospora delbruekii (exemple : référence I.4) à 0,25%
   et 0,5%

Les cellules sont ensuite incubées pendant 6 heures à 37°C.

A J2-T6h, les cellules sont récupérées (pour doser l'IGF-1) et les ARN totaux extraits. Les ARN sont reverse-transcripts et les ADN complémentaires obtenus sont analysés par la technique de PCR quantitative. Les ARNm de la protéine ribosomale S27, témoin interne de référence, sont également analysés en parallèle des ARNm d'IGF-1.

La quantification de l'incorporation de fluorescence (SYBR Green) est mesurée en continu à l'aide d'un thermocycleur. L'analyse des Ct (quantification relative) est réalisée à l'aide d'un logiciel.

Les résultats obtenus pour chacune des substances, sont présentés dans les tableaux ci-après :

| | Expression IGF-1 (%) |
|---|---|
| Fibroblastes jeunes | 199 |
| Fibroblastes âgés | 100 |
| Principe actif issu d'adzuki (ex I.1) 0.25% | 168 |
| Principe actif issu d'adzuki(ex I.1) 0.5% | 164 |
| Principe actif issu d'adzuki (ex I.1) 1% | 195 |
| Principe actif issu de Candida oleophila (ex I.2) 0.25% | 113 |
| Principe actif issu de Candida oleophila (ex I.2) 0.5% | 137 |
| Principe actif issu de Candida oleophila (ex I.2) 1% | 160 |
| Principe actif issu de Hanseniaspora opuntiae (ex I.3) 0.25% | 165 |
| Principe actif issu de Hanseniaspora opuntiae (ex I.3) à 0.5% | 235 |
| Principe actif issu de Torulospora delbruekii (ex I.4) à 0.25% | 108 |
| Principe actif issu de Torulospora delbruekii (ex I.4) à 0.5% | 142 |

On constate que les principes actifs des exemples I.1 à I.4 favorisent l'expression des ARNm codant pour l'IGF-1.

### III.2 Evaluation ex-vivo de la synthèse de IGF-1 sur explants

L'objectif de cette étude est d'évaluer la capacité de principes actifs des exemples du point I. à augmenter la synthèse des protéines IGF-1 sur des explants de peaux humaines.

L'étude a été réalisée par dosage ELISA des protéines IGF-1 sur explants de donneurs jeunes et âgés.

Le protocole opératoire est le suivant.

A J0, des punchs de 8mm de diamètre sont réalisés à partir de plastie humaine issus de donneurs jeunes et âgés et maintenus en survie dans du milieu de culture.

A J1, le milieu de culture est éliminé et remplacé par du milieu de culture contenant les principes actifs.

A J3, les sousnageants sont récupérés pour le dosage des protéines IGF-1 par ELISA.

Les résultats obtenus pour chacune des substances, sont présentés dans les tableaux ci-après :

| | % efficacité Synthèse IGF-1 |
|---|---|
| Explants témoin donneurs jeunes (26 ans) | 268 |
| Explants témoin donneurs âgés (59 ans) | 100 |
| Explants donneurs âgés + Principe actif issu d'adzuki (ex I.1) 1% | 165 |
| Explants donneurs âgés + Principe actif issu d'adzuki (ex I.1) 2% | 173 |
| Explants donneurs âgés + Principe actif issu de Candida oleophila (ex I.2) 0.5% | 106 |
| Explants donneurs âgés + Principe actif issu de Candida oleophila (ex I.2) 1% | 173 |
| Explants donneurs âgés + Principe actif issu de Hanseniaspora opuntiae (ex I.3) 0.25% | 112 |
| Explants donneurs âgés + Principe actif issu de Hanseniaspora opuntiae (ex I.3) 0.5% | 133 |
| Explants donneurs âgés + Principe actif issu de Hanseniaspora opuntiae (ex I.3) 1% | 161 |
| Explants donneurs âgés + Principe actif issu de Hanseniaspora opuntiae (ex I.3) 2% | 271 |

On constate que les principes actifs des exemples I.1 à I.3 favorisent la synthèse des protéines IGF-1 sur des explants de peau.

### IV. Evaluation de l'effet de substances sur l'IGF-1R

### Evaluation ex-vivo de l'expression de la phosphorylation d'IGF-1R sur explants humains

L'objectif de cette étude est d'évaluer la capacité de principes actifs des exemples du point I. à augmenter l'expression de la phosphorylation des IGF-1R sur des explants de peaux humaines.

L'étude a été réalisée par immunohistologie sur explants de donneurs jeunes et âgés.

Le protocole opératoire est le suivant.

A J0, des punchs de 8mm de diamètre sont réalisés à partir de plastie humaine issus de donneurs jeunes et âgés et maintenus en survie dans du milieu de culture.

A J1, le milieu de culture est éliminé et remplacé par du milieu de culture contenant les principes actifs.

A J3, les explants sont récupérés, fixés, déshydratés et inclus en paraffine. Des coupes (4*µ*m) sont réalisées à l'aide d'un microtome.

Un marquage immunohistologique de IGF-1R phosphorylé est réalisé sur les coupes de peau. La visualisation est réalisée sur microscope couplé à un système d'analyse d'images. Une analyse quantitative des images a été réalisée à l'aide du logiciel Matlab*®*.

Les résultats obtenus pour chacune des substances, sont présentés dans les tableaux ci-après :

| | Expression de phosphorylation des IGF-1R (%) |
|---|---|
| Explants témoin donneurs âgés (60 ans) | 100 |
| Explants donneurs âgés + Principe actif issu d'adzuki (ex I.1) 2% | 106 |
| Explants donneurs âgés + Principe actif issu de Candida oelophila (exI.2) 2% | 163 |
| Explants donneurs âgés + Principe actif issu de Hanseniaspora opuntiae (ex I.3) 0.5% | 160 |

On constate que les principes actifs des exemples I.1 à I.3 favorisent l'expression de la phosphorylation des IGF-1R dans la peau.

### V. Etude de la capacité d'une substance agissant sur IGF-1 et IGF1-R à réparer une fonction barrière préalablement altérée

L'objectif de cette étude est de quantifier *in vivo,* sur volontaires, l'effet restructurant au niveau des mollets d'un principe actif de l'exemple I.2 formulé à 3% en gel-émulsionné en comparaison à un placebo.

L'effet restructurant du principe actif a été étudié selon les méthodes suivantes :
- Etude de la perte insensible en eau PIE (Tewametre®)
- Etude du taux d'hydratation (Corneometre®)
- Etude de l'état de surface de la peau (Microscopie confocale)
- Etude du niveau d'hydratation (scorage visuel sur volontaires)

Cet effet a été étudié sur une peau dont la fonction barrière a été préalablement perturbée artificiellement par un détergent : le Lauryl Sulfate de Sodium (SLS) appliqué pendant 14 jours. L'actif a ensuite été appliqué pendant 14 jours et comparé au placebo.

L'application de Sodium Lauryl Sulfate perturbe de façon artificielle la fonction barrière de la couche cornée, permettant ainsi de mimer une peau présentant une fonction barrière altérée.

L'étude a été réalisée sur 22 volontaires sains, de sexe féminin d'âge compris entre 28 et 40 ans présentant une peau normale au niveau des mollets.

Les compositions utilisées sont les suivantes :
- composition selon l'invention

| | |
|---|---|
| * principe actif du point I.2 | 3% |
| * PEG-7 glyceryl cocoate | 1,2% |
| * Caprilic / Capric triglycérides | 0,8% |
| * Conservateurs | 0,7% |
| * Acrylate / C10-30 alkyl acrylate crosspolymer | 0,2% |
| * Eau | qsp 100% |

- composition placebo

| | |
|---|---|
| * PEG-7 glyceryl cocoate | 1,2% |
| * Caprilic / Capric triglycérides | 0,8% |
| * Conservateurs | 0,7% |
| * Acrylate / C10-30 alkyl acrylate crosspolymer | 0,2% |
| * Eau | qsp 100% |

Le protocole opératoire de l'étude est le suivant.

De J₋₂₈ et J₋₁₃, arrêt d'application de crème au niveau des mollets.

A J₋₁₄, détermination des zones de mesures au niveau des mollets.

Les zones de mesures sont les suivantes :
- Zone placebo
- Zone principe actif de l'exemple I.2 à 3%

Réalisation des mesures sur chacune des zones.

De J₋₁₄ et J₋₁, lavage biquotidien avec un savon irritant (SLS) sur les zones étudiées.

A J₀, détermination des zones de mesures au niveau des mollets et réalisation des mesures, évaluation et acquisition sur chacune des zones.

Entre Jo et J₆, lavage biquotidien des zones d'étude avec un savon standardisé et applications biquotidiennes du produit et du placebo.

A J₇,lavage des zones d'étude avec un savon standardisé, repérage des zones de mesure et réalisation des mesures, évaluation et acquisition sur chacune des zones.

Entre J₇ et J₁₃, lavage biquotidien des zones d'étude avec un savon standardisé et applications biquotidiennes du produit et du placebo.

A J₁₄, lavage des zones d'étude avec un savon standardisé, repérage des zones de mesure et réalisation des mesures, évaluation et acquisition sur chacune des zones.

### V.1 Etude de la perte insensible en eau

Les mesures de la PIE ont été effectuées à l'aide d'un Tewamètre®. Cet appareil est muni d'une sonde qui mesure le gradient de vapeur d'eau se mettant en place entre la surface cutanée et l'air ambiant.

Une diminution de la PIE est caractéristique d'une amélioration de la fonction barrière de la peau.

La moyenne des résultats obtenus avec le principe actif agissant sur IGF-1 IGF-1R en pourcentage de variation par rapport aux résultats obtenus avec le placebo sont les suivants :

| | **Variation / Placebo (%)** |
|---|---|
| **J7** | -9,0% |
| **J14** | -11,0% |

On constate que dans les conditions de cette étude, après 7 jours d'applications biquotidiennes, en comparaison au placebo, un principe actif agissant sur IGF-1, IGF-1R formulé à 3% en gel émulsionné diminue la PIE de 9,0%.

Cet effet se poursuit après 14 jours d'étude, pour atteindre une diminution des pertes en eau de 11,0%.

Un principe actif agissant sur IGF-1, IGF-1R, en particulier un principe actif issu de Candida oleophila tel que présenté au point I.2, permet une diminution des pertes en eau de la peau permettant ainsi de restaurer l'effet barrière d'une peau préalablement altérée.

### V.2 Etude du taux d'hydratation de la peau

Les mesures ont été effectuées à l'aide d'un Cornéomètre®. Cet appareil est muni d'une sonde qui mesure de façon quantitative et directe, la capacitance électrique de la peau, c'est-à-dire la capacité de l'eau intercellulaire du *stratum corneum* à conduire les électrons. Cette mesure est reliée directement à l'état d'hydratation de la peau.

Une augmentation du taux d'hydratation est représentative d'une amélioration de l'état d'hydratation de la peau.

La moyenne des résultats obtenus avec le principe actif agissant sur IGF-1 IGF-1R en pourcentage de variation par rapport aux résultats obtenus avec le placebo sont les suivants :

| | **Variation / Placebo (%)** |
|---|---|
| **J7** | +15,3% |
| **J14** | +26,3% |

On constate que dans les conditions de cette étude, après 7 jours d'applications biquotidiennes, en comparaison au placebo, un principe actif agissant sur IGF-1, IGF-1R formulé à 3% en gel émulsionné améliore l'hydratation d'une peau préaliablement altérée au SLS.

Cet effet se poursuit après 14 jours d'étude, pour atteindre une augmentation de 26,3% du taux d'hydratation.

Un principe actif agissant sur IGF-1, IGF-1R, en particulier un principe actif issu de Candida oleophila tel que présenté au point I.2, permet donc d'augmenter l'hydratation de la peau.

### V.3 Etude de l'état de surface de la peau

Les acquisitions (correspondant à des coupes horizontales de la peau) ont été réalisées à l'aide d'un microscope confocal sur les volontaires au niveau du *stratum corneum,* après application locale de fluorescéine.

Les images récupérées ont ensuite été évaluées visuellement par un jury entraîné composé de 4 personnes à l'aide d'une échelle de score comprenant 4 stades différents d'organisation. Chacune des photos a été codée et randomisée de façon à réaliser cette évaluation totalement en aveugle.

Plusieurs paramètres ont été pris en compte par le jury : présence de squames (quantité et taille), forme des cellules (contours hexagonaux ou arrondis), arrangement des cellules (arrangement en écaille ou nid d'abeille) selon l'échelle suivante :

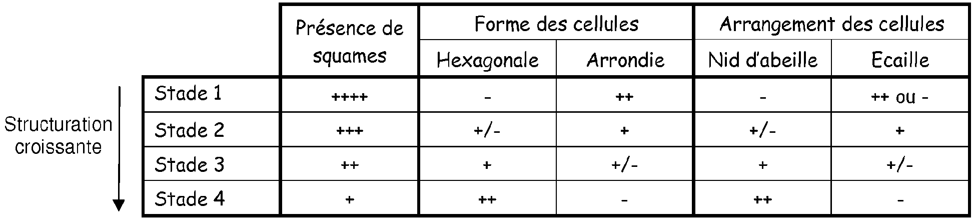

Une augmentation du stade est caractéristique d'une amélioration de l'état de surface de la peau et donc d'une amélioration de l'organisation cellulaire de la couche superficielle de la peau.

La moyenne des résultats obtenus avec le principe actif agissant sur IGF-1 IGF-1R en pourcentage de variation par rapport aux résultats obtenus avec le placebo sont les suivants :

| | **Variation / Placebo (%)** |
|---|---|
| **J7** | +15,6% |
| **J14** | +23,7% |

On constate que dans les conditions de cette étude, après 7 jours d'applications biquotidiennes, en comparaison au placebo, un principe actif agissant sur IGF-1, IGF-1R formulé à 3% en gel émulsionné favorise une amélioration de l'aspect de surface de la peau de 15,6%.

Cet effet se poursuit après 14 jours d'étude, pour atteindre une amélioration de 23,7%.

De plus 55% des personnes ont changé de score au cours de l'étude et sont passées à un score supérieure après 14 jours de traitement.

Un principe actif agissant sur IGF-1, IGF-1R, en particulier un principe actif issu de Candida oleophila tel que présenté au point I.2, permet ainsi de restaurer l'organisation cellulaire de la couche superficielle de la peau.

### V.4 Evaluation clinique de la sécheresse cutanée

Le scorage de l'état de sécheresse sur les mollets a été réalisé en aveugle en condition d'éclairage rasant standardisé par un expert entraîné.

L'évaluation visuelle a été faite sur une échelle à 4 points :
- **Stade 1** : aucun signe de sécheresse cutanée visible, aspect brillant et éclatant.
- **Stade 2** : petites particules blanches de peaux sèches, blanchiment des sillons et plis cutanés.
- **Stade 3** : apparition de petites particules blanches à l'aspect poudreux ayant tendance à se décoller dans les angles des triangles des dermatoglyples.
- **Stade 4** : larges particules blanches et sèches qui se détachent des sillons et plis cutanés sur des longueurs entières. Une rougeur et une rugosité de la peau peuvent également apparaître.

### (D'après A. J Byrne, Int J. Cosmet. Sci., 2010, 32,410-421).

Une diminution du stade est caractéristique d'une réduction de la sécheresse de la peau.

La moyenne des résultats obtenus avec le principe actif agissant sur IGF-1 IGF-1R en pourcentage de variation par rapport aux résultats obtenus avec le placebo sont les suivants :

| | **Variation / Placebo (%)** |
|---|---|
| **J7** | -5,1% |
| **J14** | -15,2% |

On constate que dans les conditions de cette étude, après 14 jours d'applications biquotidiennes, en comparaison au placebo, un principe actif agissant sur IGF-1, IGF-1R formulé à 3% en gel émulsionné réduit la sécheresse d'une peau préalablement agressée au SLS de 15,2%

Ainsi, après une altération artificielle de la fonction barrière de la peau, un principe actif agissant sur IGF-1, IGF-1R, en particulier un principe actif issu de Candida oleophila tel que présenté au point I.2 formulé à 3% en gel-émulsionné et en comparaison au placebo :
- diminue les pertes en eau et permet de restaurer l'effet barrière de la peau
- restaure l'organisation de la couche cornée visualisée par microscopie confocale
- améliore l'hydratation et réduit visuellement les signes de sécheresse cutanée

### VI. Evaluation de l'effet anti-âge d'une substance agissant sur IGF-1 et IGF1-R

L'objectif de cette étude est de quantifier *in vivo,* sur volontaires, l'effet anti-âge d'un principe actif de l'exemple I.2 formulé à 3% en gel-émulsionné en comparaison à un placebo.

L'effet anti-âge du principe actif a été étudié selon les méthodes suivantes :
- Etude du relief cutané de la patte d'oie
   ∘ projection de franges
   ∘ profilométrie optique
   ∘ scorage visuel sur photos
   ∘ scorage visuel sur volontaires
- Etude du microrelief

Les différentes méthodes ont été réalisées avant et après 56 jours d'applications biquotidiennes du principe actif et du placebo en hémi-visage. L'étude a été réalisée sur 21 volontaires sains, de sexe féminin d'âge compris entre 60 et 72 ans présentant des rides au niveau de la patte d'oie et une peau sèche au niveau du visage.

Les compositions utilisées sont les suivantes :
- composition selon l'invention

| | |
|---|---|
| * principe actif du point I.2 | 3% |
| * Isononyl isononanoate | 5% |
| * Arachidyl alcool / Behenyl Alcool / Archidyl glucoside | 3% |
| * Cetearyl alcool / Cetearyl glucoside | 2% |
| * Conservateurs | 0,7% |
| * Polyacrylamide / C13-14 Isoparaffine / Laureth-7 | 0,3% |
| * Eau | qsp 100% |

- composition placebo

| | |
|---|---|
| * Isononyl isononanoate | 5% |
| * Arachidyl alcool / Behenyl Alcool / Archidyl glucoside | 3% |
| * Cetearyl alcool / Cetearyl glucoside | 2% |
| * Conservateurs | 0,7% |
| * Polyacrylamide / C13-14 Isoparaffine / Laureth-7 | 0,3% |
| * Eau | qsp 100% |

Le protocole opératoire de l'étude est le suivant.

Entre J-14 et J-1,
- Distribution d'une crème placebo et d'un savon standardisé
- Nettoyage biquotidien du visage avec le savon.
- Applications biquotidiennes de la crème placebo sur l'ensemble du visage.

A JO,
- Nettoyage du visage avec le savon.
- Les volontaires viennent au laboratoire sans avoir appliqué de produit sur le visage le matin (ni crème, ni maquillage).
- Réalisation des mesures et évaluations

Entre J0 et J27,
- Nettoyage biquotidien du visage avec le savon.
- Applications biquotidiennes du principe actif selon l'invention et du placebo.

A J28,
- Nettoyage du visage avec le savon.
- Les volontaires viennent au laboratoire sans avoir appliqué de produit sur le visage le matin (ni crème, ni maquillage).
- Réalisation des mesures et évaluations

Entre J28 et J55,
- Nettoyage biquotidien du visage avec le savon.
- Applications biquotidiennes du produit et du placebo.

A J56,
- Nettoyage du visage avec le savon.
- Les volontaires viennent au laboratoire sans avoir appliqué de produit sur le visage le matin (ni crème, ni maquillage).
- Réalisation des mesures et évaluations

### VI.1 Evaluation de l'effet lissant

Des empreintes en polymère siliconé ont été réalisées avant et après utilisation des produits au niveau des joues.

Des acquisitions en volume de ces empreintes ont été réalisées à l'aide d'un appareil de projection de franges dédié à la mesure 3D du relief des empreintes. Les paramètres qui peuvent être étudiés sont des paramètres de rugosité en 3D :
▪ Sq : moyenne quadratique de rugosité de surface
▪ Sa : moyenne arithmétique de rugosité de surface

Une diminution de ces différents paramètres est caractéristique d'un lissage de la surface étudiée.

La moyenne des résultats obtenus avec le principe actif agissant sur IGF-1 IGF-1R en pourcentage de variation par rapport aux résultats obtenus avec le placebo sont les suivants :

| | **Variation / Placebo (%)** | |
|---|---|---|
| | J28 | J56 |
| **Paramètre Sa** | -4,2% | -5,7% |
| **Paramètre Sq** | -4,4% | -5,3% |

On constate que dans les conditions de cette étude, après 28 jours d'applications biquotidiennes, en comparaison au placebo, un principe actif agissant sur IGF-1, IGF-1R formulé à 3% en gel émulsionné améliore la rugosité de la peau en réduisant les paramètres Sa et Sq de respectivement -4,2% et -4,4%.

Cet effet se poursuit après 56 jours de traitement pour atteindre une réduction de 5,7% du paramètre Sa et de 5,3% du paramètre Sq.

Un principe actif agissant sur IGF-1, IGF-1R, en particulier un principe actif issu de Candida oleophila tel que présenté au point I.2, permet ainsi lisser le relief cutané.

### VI.2 Evaluation de l'effet anti-rides

### Etude du relief cutané par profilométrie optique

Des empreintes en polymère siliconé ont été réalisées avant et après utilisation des produits au niveau des pattes d'oie.

Un éclairage en lumière rasante à 35° génère, à la surface de l'empreinte, des ombres portées qui sont ensuite observées à l'aide d'une caméra reliée à un système informatique. On obtient ainsi une image informatique, dont l'analyse des niveaux de gris, à l'aide d'un logiciel permet d'obtenir les paramètres suivants
- Le nombre de rides
- La surface totale ridée
- La longueur totale des rides

Une diminution de ces différents paramètres est caractéristique d'un lissage du relief de la surface étudiée et d'une diminution des rides.

La moyenne des résultats obtenus avec le principe actif agissant sur IGF-1 IGF-1R en pourcentage de variation par rapport aux résultats obtenus avec le placebo sont les suivants :

| | **Variation / Placebo (%)** | |
|---|---|---|
| | J28 | J56 |
| **Nombre de rides** | +0,2% | -5,3% |
| **Longueur totale** | -6,9% | -4,4% |
| **Surface totale ridée** | -21,3% | -16,2% |

On constate que dans les conditions de cette étude, après 28 jours d'applications biquotidiennes, en comparaison au placebo, un principe actif agissant sur IGF-1, IGF-1R formulé à 3% en gel émulsionné améliore les paramètres caractéristiques du relief cutané mesuré par profilométrie optique.

Cet effet se poursuit après 56 jours de traitement.

### Etude du relief cutané par projection de franges

Des empreintes en polymère siliconé ont été réalisées avant et après utilisation des produits au niveau des joues.

Des acquisitions en volume de ces empreintes ont été réalisées à l'aide d'un appareil de projection de franges dédié à la mesure 3D du relief des empreintes. Les paramètres qui peuvent être étudiés sont des paramètres de rugosité en 3D :
▪ Sq : moyenne quadratique de rugosité de surface
▪ Sa : moyenne arithmétique de rugosité de surface

Le volume de la ride est étudié par un paramètre de volume : volume négatif c'est à dire volume inférieur à la surface de la peau

Une diminution de ces différents paramètres est caractéristique d'un lissage de la surface étudiée.

La moyenne des résultats obtenus avec le principe actif agissant sur IGF-1 IGF-1R en pourcentage de variation par rapport aux résultats obtenus avec le placebo sont les suivants :

| | **Variation / Placebo (%)** | |
|---|---|---|
| | J28 | J56 |
| **Paramètre Sa** | -4,3% | -9,8% |
| **Paramètre Sq** | -4,5% | -9,5% |
| **Volume négatif** | -11,1% | -19,6% |

On constate que dans les conditions de cette étude, après 28 jours d'applications biquotidiennes, en comparaison au placebo, un principe actif agissant sur IGF-1, IGF-1R formulé à 3% en gel émulsionné améliore le relief cutané au niveau de la patte d'oie en réduisant les paramètres de rugosité 3D et du volume négatif.

Cet effet se poursuit après 56 jours de traitement.

### Etude par scorage visuel sur photographies

Des photographies de face et de profil ont été prises dans des conditions de lumière reproductibles.

Un jury composé de 3 personnes a évalué le stade de rides de la patte d'oie de chaque photo sur une échelle de score préalablement définie.

L'échelle de score a été établie à partir d'une sélection de photos de différents grades de rides observés

L'évaluation des rides de la patte d'oie a été réalisée selon les recommandations suivantes :
- évaluation de la profondeur de la ride la plus profonde
- ne pas tenir compte du nombre de rides
- ne pas tenir compte de la longueur des rides
- ne pas tenir compte des cicatrices éventuelles

La moyenne des résultats obtenus avec le principe actif agissant sur IGF-1 IGF-1R en pourcentage de variation par rapport aux résultats obtenus avec le placebo sont les suivants :

| | **Variation / Placebo (%)** |
|---|---|
| **J28** | -4,7% |
| **J56** | -6,8% |

On constate que dans les conditions de cette étude, après 28 jours d'applications biquotidiennes, en comparaison au placebo, un principe actif agissant sur IGF-1, IGF-1R formulé à 3% en gel émulsionné réduit le stade de rides moyen de la zone de la patte d'oie après une évaluation visuelle réalisée en aveugle sur photographies.

Cet effet se poursuit après 56 jours de traitement.

### Etude par scorage visuel sur volontaires

Le scorage visuel du stade de la patte d'oie a été réalisé en aveugle par deux experts préalablement entraîné dans des conditions de lumière standardisée, sur volontaire allongé et yeux fermés.

L'échelle de scores utilisée est la même que celle utilisée pour le scorage visuel sur photographies.

La moyenne des résultats obtenus avec le principe actif agissant sur IGF-1 IGF-1R en pourcentage de variation par rapport aux résultats obtenus avec le placebo sont les suivants :

| | **Variation / Placebo (%)** |
|---|---|
| **J28** | -6,0% |
| **J56** | -7,7% |

On constate que dans les conditions de cette étude, après 28 jours d'applications biquotidiennes, en comparaison au placebo, un principe actif agissant sur IGF-1, IGF-1R formulé à 3% en gel émulsionné réduit le stade de rides moyen de la zone de la patte d'oie après une évaluation visuelle réalisée en aveugle sur volontaires. Cet effet se poursuit après 56 jours de traitement.

Ainsi, dans les conditions de cette étude, après 56 jours d'applications biquotidiennes, et en comparaison au placebo, un principe actif agissant sur IGF-1, IGF-1R, en particulier un principe actif issu de Candida oleophila tel que présenté au point I.2 formulé à 3% en émulsion, permet de lisser le microrelief cutané et présente un effet anti-rides à la fois quantifiable et visuellement perceptible directement sur volontaires.

## Revendications

1. Utilisation dans une composition cosmétique d'une quantité efficace d'au moins une substance obtenue à partir d'au moins un végétal ou d'au moins une levure, agissant sur l'IGF1 (facteur de croissance apparenté à l'insuline) et/ou l'IGF-1R (récepteur au facteur de croissance apparenté à l'insuline) des cellules cutanées, comme principe actif cosmétique destiné à prévenir ou lutter contre le vieillissement de la peau, **caractérisée en ce que** ladite substance :
- est un hydrolysat enzymatique de *Candida oleophila,*
- augmente l'expression des acides ribonucléiques messagers (ARNm) codant pour l'IGF-1 dans les fibroblastes de la peau et/ou augmente la synthèse de l'IGF-1 dans les fibroblastes de la peau et/ou augmente l'expression de la phosphorylation d'IGF-1R dans les kératinocytes épidermiques.

2. Utilisation cosmétique selon la revendication 1, comme principe actif cosmétique destiné à diminuer les rides et les ridules de la peau et/ou à améliorer la fonction barrière de la peau.

3. Procédé cosmétique pour prévenir et/ou lutter contre le vieillissement cutané, comprenant l'application topique sur la peau d'une composition comprenant au moins une substance obtenue à partir d'au moins un végétal ou d'au moins une levure agissant sur l'IGF-1 et/ou l'IGF-1R des cellules cutanées telle que définie dans l'une des revendications 1 à 2, comme principe actif cosmétique.

## Patentansprüche

1. Verwendung, in einer kosmetischen Zusammensetzung, einer wirksamen Menge mindestens einer Substanz, die aus mindestens einer Pflanze oder mindestens einer Hefe gewonnen wird und auf den IGF1 (insulinartiger Wachstumsfaktor) und/oder IGF-1R (Rezeptor des insulinartigen Wachstumsfaktors) der Hautzellen wirkt, als kosmetischer Wirkstoff, der dazu bestimmt ist, Hautalterung vorzubeugen oder zu bekämpfen, **dadurch gekennzeichnet, dass** die Substanz:
- ein enzymatisches Hydrolysat von *Candida oleophila* ist,
- die Expression von Boten-Ribonukleinsäuren (mRNA), die für IGF-1 codieren, in Hautfibroblasten erhöht und/oder die Synthese von IGF-1 in Hautfibroblasten erhöht und/oder die Expression der IGF-1 R-Phosphorylierung in epidermalen Keratinozyten erhöht.

2. Kosmetische Verwendung nach Anspruch 1 als kosmetischer Wirkstoff zur Verminderung von Falten und Fältchen der Haut und/oder zur Verbesserung der Barrierefunktion der Haut.

3. Kosmetisches Verfahren zur Vorbeugung und/oder Bekämpfung der Hautalterung, das die topische Anwendung einer Zusammensetzung auf die Haut umfasst, die als kosmetischen Wirkstoff mindestens eine Substanz enthält, die aus mindestens einer Pflanze oder mindestens einer Hefe gewonnen wird, die auf den IGF-1 und/oder IGF-1R der Hautzellen nach einem der Ansprüche 1 bis 2 einwirkt.

## Claims

1. Use in a cosmetic composition of an effective amount of at least one substance, obtained from at least one plant or at least one yeast which acts on the IGF1 (insulin-like growth factor) and/or IGF-1 R (insulin-like growth factor receptor) of skin cells, as a cosmetic active ingredient intended to prevent or fight against skin aging, **characterized in that** said substance:
- is an enzymatic hydrolyzate of *Candida oleophila,*
- increases expression of messenger ribonucleic acids (mRNAs) encoding IGF-1 in skin fibroblasts and/or increases synthesis of IGF-1 in skin fibroblasts and/or increases expression of IGF-1 R phosphorylation in epidermal keratinocytes.

2. Cosmetic use according to claim 1 as a cosmetic active ingredient intended to reduce wrinkles and fine lines of the skin and/or to improve the barrier function of the skin.

3. Cosmetic method for preventing and/or fighting cutaneous aging, comprising the topical application to the skin of a composition comprising at least one substance, obtained from at least one plant or at least one yeast which acts on the IGF-1 and/or IGF-1 R of skin cells as defined in either of claims 1 to 2, as a cosmetic active ingredient.
